# EUROPÄISCHE PATENTSCHRIFT

(11) **EP 0 555 253 B1**
(45) Veröffentlichungstag und Bekanntmachung des Hinweises auf die Patenterteilung: **13.09.1995**
(21) Anmeldenummer: 91918140.4
(22) Anmeldetag: 15.10.1991
(51) Int. Cl.: C07C 15/24, C07C 2/66, C07C 2/86

(54) **VERFAHREN ZUR HERSTELLUNG VON ISOPROPYLNAPHTHALIN**
PROCESS FOR PRODUCING ISOPROPYL NAPHTHALINE
PROCEDE DE PRODUCTION D'ISOPROPYLNAPHTALENE

(30) Priorität: 01.11.1990 DE 4034748
(43) Veröffentlichungstag der Anmeldung: 18.08.1993
(73) Patentinhaber: HOECHST AKTIENGESELLSCHAFT, 65926 Frankfurt am Main (DE)
(72) Erfinder: NEUBER, Marita, D-6000 Frankfurt am Main (DE); DETTMEIER, Udo, D-6233 Kelkheim (DE); LEUPOLD, Ernst, Ingo, D-6392 Neu-Anspach (DE)
(86) Internationale Anmeldenummer: EP9101951
(87) Internationale Veröffentlichungsnummer: WO9207810

(56) Entgegenhaltungen:
- EP-A- 0 338 292
- DE-A- 2 517 591

## Beschreibung

Die vorliegende Erfindung betrifft ein Verfahren zur Herstellung von Isopropylnaphthalin durch Alkylierung von Naphthalin mit EU-1-Zeolithen als Katalysatoren, wobei nur geringe Mengen an Diisopropylnaphthalinen entstehen.

2-(Mono)-Isopropylnaphthalin (2-MIPN) ist ein wertvolles Zwischenprodukt zur Herstellung von 2-Naphthol nach dem Hock-Verfahren. 2-Naphthol wird in der Regel durch Sulfonierung von Naphthalin in der 2-Position und anschließende Verseifung des Na-Salzes der Naphthalinsulfonsäure in einer Natriumhydroxidschmelze hergestellt. Bei diesem Prozeß fällt eine große Menge an Salzen an. Mit dem Hock-Verfahren wird dieser Nachteil weitgehend vermieden.

Außer 2-MIPN ist auch 2,6-Diisopropylnaphthalin (2,6-DIPN) ein wertvolles Zwischenprodukt für viele hochveredelte Produkte. So dient es unter anderem als Ausgangsstoff zur Herstellung von 2,6-Naphthalindicarbonsäure, 2,6-Dihydroxynaphthalin und 6-Hydroxy-2-naphthalincarbonsäure. Diese Verbindungen werden als Monomere für Hochleistungspolymere eingesetzt.

In der Praxis ist der Bedarf an 2-MIPN wegen der größeren Produktionsmenge an 2-Naphthol wesentlich höher als von 2,6-DIPN. Daher ist es wünschenswert, die Alkylierung so zu lenken, daß bei hohem Umsatz an Naphthalin viel MIPN und wenig DIPN gebildet wird.

Es ist bekannt, für die Alkylierung von Naphthalin zu MIPN und auch DIPN die verschiedensten sauren Katalysatoren einzusetzen. Bei der Alkylierung mit Friedel-Crafts-Katalysatoren, wie AlCl₃ oder BF₃, entstehen bei der Aufarbeitung Salze und der Katalysator wird zerstört. Außerdem bilden sich im Laufe der Reaktion harzartige Verbindungen, die bei der Aufarbeitung stören. Die Abtrennung der Produkte vom Katalysator ist meist aufwendig. Daher wurde seit einiger Zeit versucht, diese Art von Katalysatoren durch feste Säuren zu ersetzen.

In der DE-PS 2 644 624 wird ein Verfahren zur Herstellung von 2-MIPN unter Einsatz von P₂O₅/SiO₂ beschrieben. Dabei muß das Naphthalin in großem Überschuß eingesetzt werden, um die Bildung von mehrfach alkylierten Produkten zu vermeiden. Zudem muß das nicht umgesetzte Naphthalin abgetrennt und in die Alkylierung zurückgeführt werden. Auch bei der Verwendung von säureaktiviertem Montmorillonit (DE-OS 2 208 363) oder von perfluorierten Sulfonsäureharzen (US-PS 4 288 646) muß Naphthalin im Überschuß eingesetzt werden, um eine Bildung großer Mengen an mehrfach alkylierten Produkten zu vermeiden. Der geringe Umsetzungsgrad des Naphthalins bei all diesen Verfahren stellt einen erheblichen Nachteil dar.

Auch saure Zeolithe wurden als Katalysatoren für die Alkylierung von Naphthalin eingesetzt. In der EP-OS 338 292 wird die Umsetzung von Naphthalin mit Propylen an dealumierten Y-Zeolithen in Gegenwart von Dekahydronaphthalin beschrieben. Bei 220°C und einem Naphthalin-Umsatz von ca. 50 % beträgt dabei die Selektivität zu MIPN zwischen 68 und 75 %, zu DIPN zwischen 24 und 30 % und zu Triisopropylnaphthalinen (TIPN) zwischen 1,5 und 3 %. Bei diesem Verfahren ist das Verhältnis von MIPN/(DIPN + TIPN) mit etwa 2 bis 3 sehr gering.

In anderen Arbeiten wird die Möglichkeit der formselektiven Katalyse genutzt, um die 2-Alkyl- und 2,6-Dialkylnaphthaline herzustellen. Formselektive Katalyse bedeutet, daß die Abmessungen der an der Reaktion beteiligten Moleküle oder übergangszustände in derselben Größenordnung liegen wie die Katalysatorporen. Durch sterische Zwänge kann der Reaktionsverlauf beeinflußt werden. So kann man z.B. bei der Alkylierung von Naphthalin mit Methanol mit Zeolithen des ZSM-5-Typs mit hoher Selektivität die schlanken β-Isomeren (2-Methyl- und 2,6-Dimethylnaphthalin) erhalten (z.B. D. Fraenkel et al., J. Catal. 101 (1986) 273-283 und EP-OS 280 055). Aber auch bei diesen Verfahren ist der Naphthalin-Umsatz begrenzt, weil die Diffusion der sperrigen Moleküle durch die Zeolithporen stark behindert wird.

Bei dem Verfahren der EP-OS 317 907 wurde ein dealumierter Mordenit-Zeolith für die Alkylierung von Naphthalin mit Propylen eingesetzt. Es wurde ein Naphthalin-Umsatz von 97,3 % erzielt. Die Ausbeute an DIPN betrug 68 %, wovon 50 % aus 2,6-DIPN bestanden. Über die Ausbeute an MIPN und die Zusammensetzung dieser Fraktion wurden keine Aussagen getroffen. Auch in dem Verfahren nach der WO 90/03961 wird ein dealumierter Mordenit-Zeolith zur selektiven Herstellung von 2,6-DIPN verwendet. Es wird ausgeführt, daß durch diesen Zeolithtyp der Anteil von 2,6-DIPN an der DIPN-Fraktion größer als der Anteil im thermodynamischen Gleichgewicht und das Verhältnis von 2,6-/2,7-DIPN größer als 1,2 werden. So soll beispielsweise bei 27 %igem Umsatz an Naphthalin der Anteil von 2,6-DIPN 70 %, das Verhältnis von 2,6-/2,7-DIPN 3,0 und das Verhältnis von MIPN/(DIPN + TIPN) 5,1 betragen. Steigt der Umsatz auf 78 %, nimmt der Anteil von 2,6-DIPN auf 62 %, das Verhältnis von 2,6-/2,7-DIPN auf 2,6 und das Verhältnis von MIPN/(DIPN + TIPN) auf 1,1 ab. Über die Zusammensetzung der MIPN-Fraktion werden keine Angaben gemacht.

Diese Beispiele zeigen, daß Mordenit-Katalysatoren bei steigendem Umsatz an Naphthalin die Bildung von MIPN gegenüber DIPN stark zurückdrängen.

Aufgabe der vorliegenden Erfindung war es, ein Verfahren zur Herstellung von MIPN durch Alkylierung von Naphthalin zur Verfügung zu stellen, das bei hohem Umsatz an Naphthalin möglichst wenig DIPN liefert.

Dies wird gemäß der Erfindung durch den Einsatz von EU-1-Zeolithen als Katalysatoren erreicht. Dabei werden mit hoher Selektivität einfach alkylierte Produkte und nur in untergeordnetem Maß zweifach alkylierte Verbindungen gebildet. Noch höher alkylierte Verbindungen, wie TIPN entstehen bei diesem Verfahren kaum.

Gegenstand der Erfindung ist nun ein Verfahren zur Herstellung von Isopropylnaphthalin durch Alkylierung von Naphthalin mit Hilfe von Katalysatoren, das dadurch gekennzeichnet ist, daß als Katalysatoren EU-1-Zeolithe eingesetzt werden.

Zeolithe sind kristalline Alumosilikate. Si- und Al-Atome sind tetraedrisch von O-Atomen umgeben. Die Tetraeder sind über gemeinsame O-Atome verknüpft und bilden eine Kristallstruktur, die von definierten Poren und Hohlräumen durchzogen ist (vgl. D.W. Breck, Zeolite Molecular Sieves, John Wiley & Sons, (1974) S. 29-185). Die erfindungsgemäß verwendeten Zeolithe vom EU-1-Typ sind durch ein typisches Röntgenbeugungsdiffraktogramm, das in der EP-OS 42 226 angegeben ist, charakterisiert. Die Poren dieses Zeoliths werden durch 10 O-Atome begrenzt und haben eine Weite von 0,41 x 0,58 nm. Sie besitzen seitliche Erweiterungen, die von 12 O-Atomen gebildet werden. Diese Ausbuchtungen sind 0,58 x 0,68 nm weit und 0,81 nm tief (N.A. Briscoe et al., Zeolites 8, (1988) 74-76).

Es ist überraschend, daß gerade mit diesem Zeolithtyp die gewünschte hohe Selektivität zu den MIPN und die geringe Bildungsgeschwindigkeit von DIPN erzielt werden kann, da sowohl der in der EP-OS 317 907 beschriebene Einsatz von Mordenit-Katalysatoren, mit im Vergleich zu EU-1-Zeolithen größeren Poren von 0,67 x 0,70 nm, als auch der Einsatz von Zeolithen vom Typ ZSM-5, mit etwas engeren Poren von 0,52 x 0,55 nm und 0,54 x 0,56 nm, zu einer weit stärkeren Bildung von DIPN führt.

Die unterschiedlichen Selektivitäten dieser Katalysatoren werden in den Beispielen verglichen.

Die erfindungsgemäß verwendeten EU-1-Zeolithe können nach bekannten Verfahren durch hydrothermale Synthese hergestellt werden (z.B. EP-OS 42 226). Als Templat werden alkylierte Polymethylendiamine, bevorzugt N,N,N,N',N',N'-Hexamethyl-1,6-hexamethylendiammoniumbromid (Hexamethoniumbromid), verwendet. Das SiO₂/Al₂O₃-Verhältnis kann durch direkte Synthese in einem Bereich von 10 bis 150 eingestellt werden. Durch nachträgliche Dealuminierung können allerdings auch höhere SiO₂/Al₂O₃-Verhältnisse erreicht werden. Der Aluminiumgehalt kann dabei auf verschiedene Weise verringert werden. Einige Methoden sind z.B. in J. Scherzer, Catalytic Materials, Relationship between Structure and Reactivity, ACS Symp. Ser. 248 (1984) 175-200, beschrieben. Für das erfindungsgemäße Verfahren sind EU-1-Zeolithe mit SiO₂/Al₂O₃-Verhältnissen zwischen 15 und 200, bevorzugt zwischen 20 und 100, besonders geeignet.

Nach der Kristallisation wird der Zeolith abfiltriert, gewaschen, getrocknet und anschließend in oxidierender Atmosphäre, bevorzugt an Luft, calciniert, um das organische Templat aus den Poren zu entfernen.

Um den Zeolith in eine katalytisch aktive Form zu überführen, werden die eventuell vorhandenen Na⁺-Ionen durch Ionenaustausch gegen zwei- oder dreiwertige Ionen der Erdalkalimetalle oder Seltenen Erdmetalle der Ordnungszahl 57 bis 71 oder gegen Ammonium-Ionen oder Protonen ausgetauscht. Der Ionenaustausch mit NH₄⁺ oder H⁺ ist besonders bevorzugt. Dabei ist es zweckmäßig, daß mindestens 50 %, vorzugsweise mindestens 90 % der Gitterladungen durch die genannten anderen Ionen kompensiert werden. Der Zeolith wird dann durch Dehydratisierung (und Deammonisierung bei NH₄⁺-Formen) bei 200 bis 800°C, bevorzugt bei 400 und 550°C in die katalytisch aktive Form übergeführt.

Die Zeolithe können für die erfindungsgemäße Verwendung vorteilhaft mit Hilfe von Bindemitteln in eine geeignete Anwendungsform, z.B. in Strangform gebracht werden. Als Bindemittel sind vor allem Oxide, Hydroxide oder Hydroxychloride des Aluminiums und die Oxide des Siliziums, Titans und Zirkons sowie Tonmaterialien geeignet.

Die Alkylierungsreaktion kann in der Gasphase, vorzugsweise aber in der Flüssigphase durchgeführt werden. Als Alkylierungsmittel können beispielsweise i-Propylbromid und -chlorid, Propylen und i-Propanol eingesetzt werden. Bei der Gasphasenreaktion ist es bevorzugt Naphthalin mit Propylen oder i-Propanol umzusetzen. Bei der Alkylierung in der Flüssigphase ist die Verwendung von Propylen bevorzugt. Die Reaktionstemperatur liegt zweckmäßig zwischen 100 und 500°C, bevorzugt zwischen 150 und 300°C. Ein erhöhter Druck ist günstig für den Verlauf der Alkylierung, insbesondere, wenn mit Propylen alkyliert wird. Die Reaktion kann bei Unterdruck, Atmosphärendruck oder höherem Druck, z.B. bis etwa 100 bar, bevorzugt zwischen 2 und 20 bar, durchgeführt werden.

Die Alkylierung in der Flüssigphase kann in allen geeigneten Apparaturen durchgeführt werden, am einfachsten in einem Rührkessel mit pulverförmigem, in geschmolzenem Naphthalin suspendiertem Katalysator. Das Alkylierungsmittel wird dann bei Reaktionstemperatur durch die Suspension geleitet oder bis zum gewünschten Druck aufgepreßt. Die Reaktion wird bevorzugt ohne Lösungsmittel durchgeführt. Es können aber auch gegenüber den Reaktionsteilnehmern und Katalysatoren inerte Lösungsmittel, wie höhersiedende Paraffine oder Naphthene, anwesend sein. Zur Erzielung des Reaktionsdruckes können auch inerte Gase wie Stickstoff verwendet werden.

Das Verfahren kann kontinuierlich oder diskontinuierlich durchgeführt werden.

Bezogen auf die Masse an eingesetztem Naphthalin ist es bei diskontinuierlicher Arbeitsweise günstig, 0,5 bis 50 Gew.-% Katalysator, bevorzugt 1 bis 10 Gew.-%, einzusetzen. Die Reaktionsdauer kann je nach Reaktionsbedingungen und gewünschtem Umsatz zwischen etwa einer halben Stunde und mehreren Tagen, insbesonder zwischen 2 und 10 h, betragen. Nach erfolgter Reaktion kann der Zeolith auf einfache Weise, z.B. durch Filtration, aus dem Reaktionsgemisch abgetrennt werden.

Für die Durchführung der Reaktion in der Gasphase können prinzipiell alle für Gasphasenreaktionen geeigneten Apparaturen verwendet werden. Technisch am einfachsten ist ein Festbett-Strömungsreaktor zu handhaben. Der Katalysator kann dabei in Form von Pellets in den Reaktor eingebaut werden. Für die Herstellung der Pellets kann der Zeolith für sich oder zusammen mit einem Bindemittel wie Al₂O₃ oder SiO₂ verpreßt werden. Das Naphthalin kann in geschmolzenem Zustand oder in einem inerten Lösungsmittel gelöst in den Reaktor eindosiert und vor dem Katalysatorbett verdampft oder aber schon in gasförmigem Zustand in den Reaktor geleitet werden. i-Propanol kann ebenso wie Naphthalin dosiert werden.

Propylen wird gasförmig eingeleitet. Die Reaktionsteilnehmer können allein oder im Gemisch mit einem hinsichtlich der Reaktion inerten Gas, wie Wasserstoff oder Stickstoff, eingesetzt werden. Die Reaktionsprodukte werden nach dem Verlassen des Reaktors kondensiert.

Das molare Verhältnis von Naphthalin zum Alkylierungsmittel liegt zweckmäßig im Bereich von 0,1 bis 10, bevorzugt von 0,5 bis 2.

Die Verweilzeit der Reaktionsteilnehmer liegt im allgemeinen zwischen 0,05 und 20 s, bevorzugt zwischen 1 und 10 s. Die Belastung (LHSV = liquid hourly space velocity = ml Einsatz pro ml Katalysatorvolumen und Stunde) kann vorzugsweise im Bereich von 0,1 bis 5 h⁻¹ eingestellt werden, wobei der Bereich von 0,5 bis 2 h⁻¹ besonders günstig ist.

Der Katalysator behält seine Aktivität über lange Zeit und ist mehrfach für die Reaktion einsetzbar. Falls er desaktiviert ist, kann er durch Calcinieren in oxidierender Atmosphäre, bevorzugt an Luft, bei 350 bis 800°C, bevorzugt bei 500 bis 600°C, wieder regeneriert werden.

Das Produktgemisch kann zunächst durch Destillation in nicht umgesetztes Naphthalin, MIPN, DIPN und TIPN aufgetrennt werden. Aus der MIPN-Fraktion kann gewünschtenfalls 2-MIPN durch Kristallisation, eventuell aus einem Lösungsmittel wie Methanol oder i-Propanol, abgetrennt werden (siehe z.B. DE-OS 2 517 591). Das an 1-MIPN angereicherte Filtrat kann durch Isomerisierung an verschiedenen Zeolithen wieder in ein an 2-MIPN reiches Gemisch übergeführt werden (siehe z.B. US-PS 4 026 959). 2,6-DIPN kann ebenfalls durch Kristallisation aus der DIPN-Fraktion abgetrennt werden (siehe z.B.

EP-PS 216 009). Eine adsorptive Abtrennung ist z.B. in JP-OS 01 199 921 beschrieben. Der Rest der von 2,6-DIPN weitgehend befreiten DIPN-Fraktion kann zusammen mit Naphthalin durch Transalkylierung wieder zu MIPN umgesetzt werden.

Die rohen 2-MIPN und 2,6-DIPN können durch übliche Aufarbeitungsverfahren weiter bis zum gewünschten Grad gereinigt werden.

### Beispiele

Die verwendeten EU-1-Zeolithe wurden nach Vorschriften aus der Literatur mit unterschiedlichen SiO₂/Al₂O₃-Verhältnissen synthetisiert (EP-PS 42 226, US-PS 4 537 754, G.W. Dodwell et al., Zeolites 5 (1985) 153-157). Alle Zeolithe werden in der Protonenform (Ionenaustausch mit NH₄NO₃-Lösung und anschließendes Calcinieren) eingesetzt.

1 und 2) Die Beispiele und die Vergleichsbeispiele V1 bis V3 wurden in einem Rührkessel durchgeführt. Der Zeolith wurde vor der Reaktion 1 h lang bei 300 °C getrocknet und dann pulverförmig in 128 g geschmolzenem Naphthalin suspendiert. Propylen wurde bei Atmosphärendruck mit 6,5 l/h (bzw. mit 12 l/h in Beispiel 2) durch die Suspension geleitet. Die Reaktionstemperatur betrug 200°C. Die Versuchsergebnisse sind in Tabelle 1 zusammengestellt.

**Tabelle 1**

| Beispiel | V1 | V2 | V3 | 1 | 2 |
|---|---|---|---|---|---|
| Zeolith | ZSM-5 | MORDENIT | | EU-1 | EU-1 |
| Umsatz an Naphthalin, mol-% | 23 | 22 | 76 | 23 | 75 |

| Selektivitäten, mol-% | | | | | |
|---|---|---|---|---|---|
| MIPN | 88 | 89 | 54 | 95 | 62 |
| DIPN | 10 | 11 | 44 | 5 | 32 |
| TIPN | 2 | 0 | 2 | 0 | 6 |
| MIPN/(DIPN+TIPN) | 7,3 | 8,1 | 1,2 | 19,0 | 1,6 |

Ein Vergleich von V1, V2 und Beispiel 1 zeigt, daß die Selektivität, mit der MIPN gebildet wird, bei nicht zu hohen Umsätzen an Naphthalin durch den Einsatz von EU-1-Zeolithen stark gesteigert wird und auch bei hohen Umsätzen (Vergleich von V3 und Beispiel 2) deutlich bessere Ergebnisse liefert.

3 bis 5) Die Beispiele und die Vergleichsbeispiele V4 bis V7 wurden in einem Festbett-Strömungsreaktor bei Atmosphärendruck durchgeführt. Der Katalysator wurde in Form von Strängen eingesetzt. EU-1 wurde mit 21 Gew.-% SiO₂ als Bindemittel verpreßt und Mordenit mit 40 % Al₂O₃. ZSM-5 wurde ohne Bindemittel zu Tabletten verpreßt. Naphthalin wurde in geschmolzenem Zustand in den Reaktor eindosiert. Propylen wurde in zweifach molarer Menge eingesetzt. Die Reaktionsprodukte wurden nach Verlassen des Reaktors in bestimmten Zeitabständen kondensiert und gaschromatographisch analysiert. Die Ergebnisse (mit T = 280°C, LHSV = 0,5 h⁻¹ (bez. auf Naphthalin), Katalysatorvolumen = 25 ml, Mengenverhältnis von Naphthalin zu Propylen = 1 : 2) sind in Tabelle 2 zusammengefaßt.

**Tabelle 2**

| Beispiel | 3 | 4 | 5 | V4 | V5 | V6 | V7 |
|---|---|---|---|---|---|---|---|
| Zeolith | EU-1 | | | MORDENIT | | | ZSM-5 |
| Zeit, h | 1,1 | 2,1 | 5,1 | 1,2 | 3,1 | 7,1 | 2,1 |
| Umsatz an Naphthalin, mol-% | 39 | 30 | 28 | 36 | 23 | 9 | 3 |

| Selektivitäten, mol-% | | | | | | | |
|---|---|---|---|---|---|---|---|
| MIPN | 78 | 83 | 84 | 64 | 71 | 85 | 53 |
| DIPN | 18 | 15 | 14 | 33 | 27 | 15 | 35 |
| TIPN | 2 | 1 | 1 | 3 | 2 | 0 | 12 |
| MIPN/(DIPN+TIPN) | 3,9 | 5,2 | 5,6 | 1,8 | 2,4 | 5,7 | 1,1 |

Ein Vergleich der Beispiele 3 bis 5 mit den Vergleichsbeispielen V4 bis V7 zeigt, daß bei Einsatz von EU-1-Zeolithen nach gleichen Zeiten deutlich höhere Umsätze an Naphthalin erzielt werden, wobei dieser Zeolithtyp auch bezüglich der Selektivität, mit der MIPN gebildet wird, deutlich überlegen ist. Außerdem zeigt sich bei den EU-1-Zeolithen eine geringere Desaktivierung nach längeren Zeiten.

## Patentansprüche

1. Verfahren zur Herstellung von Isopropylnaphthalin durch Alkylierung von Naphthalin mit Hilfe von Katalysatoren, dadurch gekennzeichnet, daß als Katalysatoren EU-1-Zeolithe verwendet werden.

2. Verfahren nach Anspruch 1, dadurch gekennzeichnet, daß der EU-1-Zeolith ein SiO₂/Al₂O₃-Verhältnis von 15 bis 200, bevorzugt von 20 bis 100, aufweist.

3. Verfahren nach Anspruch 1 oder 2, dadurch gekennzeichnet, daß in den EU-1-Zeolithen mindestens 50 %, bevorzugt mindestens 90 %, der Gitterladungen durch Protonen, Ammoniumionen, Erdalkalimetallionen und/oder Ionen der Seltenen Erdmetalle der Ordnungszahl 57 bis 71 im Periodischen System der Elemente kompensiert werden.

4. Verfahren nach einem oder mehreren der Ansprüche 1 bis 3, dadurch gekennzeichnet, daß die Reaktion in der flüssigen Phase durchgeführt wird.

5. Verfahren nach einem oder mehreren der Ansprüche 1 bis 4, dadurch gekennzeichnet, daß die Reaktion bei Temperaturen von 100 bis 500°C, bevorzugt von 150 bis 300°C, durchgeführt wird.

6. Verfahren nach einem oder mehreren der Ansprüche 1 bis 5, dadurch gekennzeichnet, daß die Reaktion bei einem Druck bis etwa 100 bar, bevorzugt bei 2 bis 20 bar, durchgeführt wird.

7. Verfahren nach einem oder mehreren der Ansprüche 1 bis 6, dadurch gekennzeichnet, daß als Alkylierungsmittel i-Propylbromid, i-Propylchlorid, Propylen oder i-Propanol eingesetzt werden.

8. Verfahren nach einem oder mehreren der Ansprüche 1 bis 7, dadurch gekennzeichnet, daß das Naphthalin im molaren Verhältnis von 0,1 bis 10, bevorzugt 0,5 bis 2, bezogen auf das Alkylierungsmittel, eingesetzt wird.

9. Verfahren nach einem oder mehreren der Ansprüche 1 bis 8, dadurch gekennzeichnet, daß bei diskontinuierlicher Arbeitsweise bezogen auf die Masse an eingesetztem Naphthalin, 0,5 bis 50 Gew.-%, bevorzugt 1 bis 10 Gew.-%, Katalysator eingesetzt werden.

## Claims

1. A process for the preparation of isopropylnaphthalene by alkylation of naphthalene with the aid of catalysts, which comprises using EU-1 zeolites as catalysts.

2. The process as claimed in claim 1, wherein the EU-1 zeolite has an SiO₂/Al₂O₃ ratio of 15 to 200, preferably of 20 to 100.

3. The process as claimed in claim 1 or 2, wherein at least 50%, preferably at least 90%, of the lattice charges in the EU-1 zeolites are compensated by protons, ammonium ions, alkaline earth metal ions and/or ions of the rare earth metals of atomic number 57 to 71 in the Periodic Table of the Elements.

4. The process as claimed in one or more of claims 1 to 3, wherein the reaction is carried out in the liquid phase.

5. The process as claimed in one or more of claims 1 to 4, wherein the reaction is carried out at temperatures from 100 to 500°C, preferably from 150 to 300°C.

6. The process as claimed in one or more of claims 1 to 5, wherein the reaction is carried out at a pressure of up to about 100 bar, preferably at 2 to 20 bar.

7. The process as claimed in one or more of claims 1 to 6, wherein isopropyl bromide, isopropyl chloride, propylene or isopropanol is used as the alkylating agent.

8. The process as claimed in one or more of claims 1 to 7, wherein the naphthalene is used in a molar ratio of 0.1 to 10, preferably 0.5 to 2, relative to the alkylating agent.

9. The process as claimed in one or more of claims 1 to 8, wherein 0.5 to 50% by weight, preferably 1 to 10% by weight, of catalyst is used in the batch procedure, based on the weight of naphthalene used.

## Revendications

1. Procédé de préparation d'isopropylnaphtalène par alkylation du naphtalène avec l'aide de catalyseurs, caractérisé en ce que, en tant que catalyseurs, on utilise des zéolithes de type EU-1.

2. Procédé selon la revendication 1, caractérisé en ce que la zéolithe de type EU-1 présente un rapport SiO₂/Al₂O₃ compris entre 15 et 200, de préférence entre 20 et 100.

3. Procédé selon la revendication 1 ou 2, caractérisé en ce que, dans les zéolithes de type EU-1, on compense au moins 50 %, de préférence au moins 90 %, des charges du réseau par des protons, des ions ammonium, des ions de métaux alcalino-terreux et/ou des ions de métaux de terres rares de numéros atomiques allant de 57 à 71 dans le Système Périodique des Eléments.

4. Procédé selon une ou plusieurs des revendications 1 à 3, caractérisé en ce que la réaction est réalisée en phase liquide.

5. Procédé selon une ou plusieurs des revendications 1 à 4, caractérisé en ce que la réaction est réalisée à des températures allant de 100 à 500°C, de préférence de 150 à 300°C.

6. Procédé selon une ou plusieurs des revendications 1 à 5, caractérisé en ce que la réaction est réalisée à une pression allant jusqu'à environ 100 bars, de préférence allant de 2 à 20 bars.

7. Procédé selon une ou plusieurs des revendications 1 à 6, caractérisé en ce que, en tant qu'agent d'alkylation, on utilise du bromure de i-propyle, du chlorure de i-propyle, du propylène ou du i-propanol.

8. Procédé selon une ou plusieurs des revendications 1 à 7, caractérisé en ce que le naphtalène est utilisé dans des rapports molaires allant de 0,1 à 10, de préférence de 0,5 à 2, par rapport à l'agent d'alkylation.

9. Procédé selon une ou plusieurs des revendications 1 à 8, caractérisé en ce que, lors d'un mode opératoire en discontinu, on utilise de 0,5 à 50 % en poids de catalyseur, de préférence de 1 à 10 % en poids, par rapport à la masse de naphtalène utilisée.
